# EUROPÄISCHE PATENTSCHRIFT

(11) **EP 0 415 885 B1**
(45) Veröffentlichungstag und Bekanntmachung des Hinweises auf die Patenterteilung: **15.03.1995**
(21) Anmeldenummer: 90810628.9
(22) Anmeldetag: 21.08.1990
(51) Int. Cl.: C07D 493/20, B41M 5/145, B41M 5/30

(54) **Bis-Phthalidlaktone, Verfahren zu deren Herstellung und ihre Verwendung in Aufzeichnungsmaterialien**
Bis-Phthalideactones, process for their preparation, and their use in recording materials
Bis-Phthalide-lactones, procédé pour leur préparation et leur utilisation pour des matériaux d'enregistrement

(30) Priorität: 29.08.1989 CH 3126/89
(43) Veröffentlichungstag der Anmeldung: 06.03.1991
(73) Patentinhaber: CIBA-GEIGY AG, 4002 Basel (CH)
(72) Erfinder: Zink, Rudolf, CH-4106 Therwil (CH); Fletcher, Ian John, Dr., CH-4312 Magden (CH)

(56) Entgegenhaltungen:
- EP-A- 0 112 710
- EP-A- 0 156 250
- EP-A- 0 176 161
- DE-A- 2 937 525
- DE-A- 3 618 562
- DE-B- 1 277 862

## Beschreibung

Die vorliegende Erfindung betrifft Bis-Phthalidlaktone, Verfahren zu ihrer Herstellung und ihre Verwendung in druck- oder wärmeempfindlichen Aufzeichnungsmaterialien.

In den Druckschriften DE-A-3,618,562, EP-A-0,122,710, EP-A-0,156,250, EP-A-0,176,161 und DE-A-2,937,525 werden Phthalide offenbart, dis sich für für druck- und wärmempfindliche Aufzeichnungsmaterialien eignen.

Die erfindungsgemässen Bis-Phthalidlaktone entsprechen der allgemeinen Formel 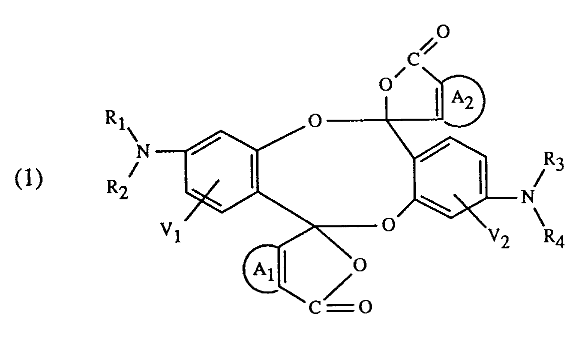
worin R₁, R₂, R₃ und R₄, unabhängig voneinander, je Wasserstoff, unsubstituiertes oder durch Halogen, Hydroxy, Cyano, Tetrahydrofuryl oder Niederalkoxy substituiertes Alkyl mit höchstens 12 Kohlenstoffatomen, Acyl mit 1 bis 12 Kohlenstoffatomen, Cycloalkyl mit 5 bis 10 Kohlenstoffatomen oder unsubstituiertes oder durch Halogen, Cyano, Nitro, Trifluormethyl, Niederalkyl, Niederalkoxy, Niederalkoxycarbonyl, -NX'X'' oder 4-NX'X''-phenylamino substituiertes Aralkyl oder Aryl, worin X' und X'', unabhängig voneinander, Wasserstoff, Niederalkyl, Cyclohexyl, Benzyl oder Phenyl darstellen, oder die Substituentenpaare (R₁ und R₂) und (R₃ und R₄) je zusammen mit dem gemeinsamen Stickstoffatom einen fünf- oder sechsgliedrigen, vorzugsweise gesättigten, heterocyclischen Rest, V₁ und V₂ Wasserstoff, Halogen, Niederalkyl, C₁-C₁₂-Alkoxy, C₁-C₁₂-Acyloxy, Benzyl, Phenyl, Benzyloxy, Phenyloxy, durch Halogen, Cyano, Niederalkyl oder Niederalkoxy substituiertes Benzyl oder Benzyloxy, oder die Gruppe -NT₁T₂, T₁ und T₂, unabhängig voneinander, je Wasserstoff, Niederalkyl, C₅-C₁₀-Cycloalkyl, unsubstituiertes oder durch Halogen, Cyano, Niederalkyl oder Niederalkoxy substituiertes Benzyl, oder Acyl mit 1 bis 8 Kohlenstoffatomen und T₁ auch unsubstituiertes oder durch Halogen, Cyano, Niederalkyl oder Niederalkoxy substituiertes Phenyl, bedeuten und die Ringe A₁ und A₂, unabhängig voneinander, einen aromatischen oder heterocyclischen Rest mit 6 Ringatomen darstellen, die einen aromatischen annellierten Ring aufweisen können, wobei sowohl die Ringe A₁ und A₂ als auch die annellierten Ringe substituiert sein können.

Stellen die Substituenten R₁, R₂, R₃ und R₄ Alkylgruppen dar, so können sie geradkettig oder verzweigt sein. Beispiele solcher Alkylreste sind Methyl, Ethyl, n-Propyl, Isopropyl, n-Butyl, 1-Methylbutyl, sek.-Butyl, tert.-Butyl, Amyl, Isoamyl, n-Hexyl, 2-Ethyl-hexyl, n-Heptyl, n-Octyl, Isooctyl, 1,1,3,3-Tetramethylbutyl, n-Nonyl, Isononyl, 3-Ethylheptyl, Decyl oder n-Dodecyl.

Sind die Alkylreste in R₁, R₂, R₃ und R₄ substituiert, so handelt es sich vor allem um Cyanoalkyl, Halogenalkyl, Hydroxyalkyl, Niederalkoxyalkyl jeweils vorzugsweise mit insgesamt 2 bis 8 Kohlenstoffatomen, wie z.B. 2-Cyanoethyl, 2-Chlorethyl, 2-Chlorpropyl, 2-Hydroxyethyl, 3-Hydroxypropyl, 2-Methoxyethyl, 2-Ethoxyethyl, 2,3-Dihydroxypropyl, 2-Hydroxy-2-chlorpropyl, 3-Methoxypropyl, 4-Methoxybutyl oder 4-Propoxybutyl sowie auch Tetrahydrofurfuryl.

Beispiele für Cycloalkyl in der Bedeutung der R-Reste, T₁ und T₂ sind Cyclopentyl, Cycloheptyl oder vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Als Aralkyl können R₁, R₂, R₃ und R₄ Phenethyl, Phenylisopropyl oder vor allem Benzyl sein. Als Aryl stellen die R-Reste besonders Naphthyl oder in erster Linie Phenyl dar.

Bevorzugte Substituenten in der Aralkyl- und Arylgruppe der R-Reste sind z.B. Halogen, Cyano, Methyl, Trifluormethyl, Methoxy oder Carbomethoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorphenyl, Methoxyphenyl, Trifluormethylphenyl, 2,6-Dimethylphenyl oder Carbomethoxyphenyl.

Wenn die Substituentenpaare (R₁ und R₂) und (R₃ und R₄) jeweils zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino, z.B. N-Methylpiperazino oder N-Phenylpiperazino. Bevorzugte gesättigte heterocyclische Reste für -NR₁R₂ und -NR₃R₄ sind Pyrrolidino, Piperidino oder Morpholino.

-NR₁R₂ und -NR₃R₄ sind vorzugsweise identisch.

Die Substituenten R₁, R₂, R₃ und R₄ sind vorzugsweise Cyclohexyl, Tolyl, Benzyl, Cyano-Niederalkyl, z.B. 2-Cyanoethyl oder in erster Linie Niederalkyl, wie z.B. Methyl, Ethyl, n-Propyl, Isopropyl, n-Butyl oder Isoamyl. -NR₁R₂ und -NR₃R₄ sind bevorzugt auch Pyrrolidino, N-Niederalkyl-N-tetrahydrofurfurylamino, 4-Diniederalkylaminophenylamino oder 4-(4'-Phenylamino-phenylamino)-phenylamino.

Der Acyloxyrest in V₁ und V₂ ist beispielsweise Formyloxy, Niederalkylcarbonyloxy wie z.B. Acetyloxy oder Propionyloxy, oder Benzoyloxy. Als C₁-C₁₂-Alkoxyrest können V₁ und V₂ eine geradkettige oder verzweigte Gruppe sein, wie z.B. Methoxy, Ethoxy, Isopropoxy, n-Butoxy, tert-Butoxy, Amyloxy, 1,1,3,3-Tetramethylbutoxy, n-Hexyloxy, n-Octyloxy oder Dodecyloxy.

V₁ und V₂ sind vorteilhafterweise Wasserstoff, Halogen, Niederalkyl, wie z.B. Methyl, Benzyloxy, C₁-C₈-Alkoxy, in erster Linie Niederalkoxy, wie z.B. Methoxy, Ethoxy, Isopropoxy oder tert.Butoxy, oder die Gruppe -NT₁T₂ sein, wobei von den Resten T₁ und T₂ eines vorzugsweise C₁-C₈-Acyl oder Niederalkyl und das andere Wasserstoff oder Niederalkyl ist. Der Acylrest ist in diesem Falle besonders Niederalkylcarbonyl, wie z.B. Acetyl oder Propionyl. Vorzugsweise sind V₁ und V₂ Acetylamino, Dimethylamino, Diethylamino, Benzyloxy oder besonders Niederalkoxy und vor allem Ethoxy oder Wasserstoff.

V₁ und V₂ befinden sich vorzugsweise in Ortho-Stellung zu -NR₁R₂ bzw. -NR₃R₄.

Als 6-gliedriger aromatischer Ring stellen A₁ und A₂ vorzugsweise einen Benzolring dar, der unsubstituiert oder durch Halogen, Cyano, Nitro, Niederalkyl, Niederalkoxy, Niederalkylthio, Niederalkylcarbonyl, Niederalkoxycarbonyl, Amino, Niederalkylamino, Diniederalkylamino oder Niederalkylcarbonylamino substituiert ist. Als 6-gliedriger heterocyclischer Ring stellen A₁ und A₂ insbesondere einen stickstoffhaltigen Hetercyclus mit aromatischem Charakter, wie z.B. einen Pyridin- oder Pyrazinring dar. Die Ringe A₁ und A₂ können auch einen annellierten aromatischen Ring, vorzugsweise einen Benzolring enthalten und stellen somit z.B. einen Naphthalin-, Chinolin- oder Chinoxalinring dar.

Die durch A₁ und A₂ wiedergegebenen bevorzugten 6-gliedrigen aromatischen oder heterocyclischen Reste sind der 2,3-Pyridino-, 3,4-Pyridino-, 2,3-Pyrazino-, 2,3-Chinoxalino-, 1,2-Naphthalino, 2,3-Naphthalino- oder 1,2-Benzorest, der unsubstituiert oder durch Halogen, wie Chlor oder Brom, Nitro, Niederalkyl, Niederalkoxy, Niederalkylthio oder eine wie vorstehend definiert gegebenenfalls substiutierte Aminogruppe substituiert ist, wobei der unsubstituierte oder durch 4 Chloratome substituierte 1,2-Benzorest besonders bevorzugt ist.

"Acyl" ist besonders Formyl, Niederalkylcarbonyl, wie z.B. Acetyl oder Propionyl, oder Benzoyl. Weitere Acylreste können Niederalkylsulfonyl, wie z.B. Methylsulfonyl oder Ethylsulfonyl sowie Phenylsulfonyl sein. Benzoyl und Phenylsulfonyl können durch Halogen, Methyl, Methoxy oder Ethoxy substituiert sein.

Niederalkyl, Niederalkoxy und Niederalkylthio stellen solche Gruppen oder Gruppenbestandteile dar, die 1 bis 6, insbesondere 1 bis 3 Kohlenstoffatome aufweisen. Beispiele für derartige Gruppen sind Methyl, Ethyl, n-Propyl, Isopropyl, n-Butyl, sek.Butyl, tert.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.

Halogen bedeutet beispielsweise Fluor, Brom oder vorzugsweise Chlor.

Besonders wichtige Bis-Phthalidlaktonverbindungen entsprechen der Formel 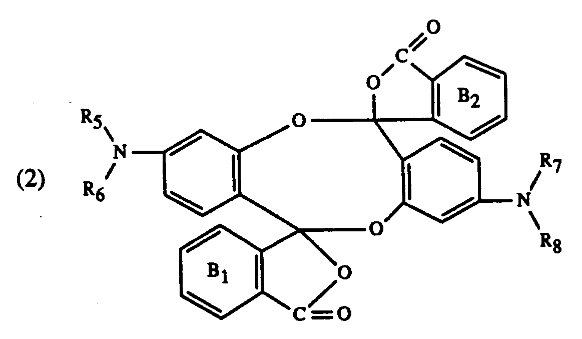
worin R₅, R₆, R₇ und R₈, unabhängig voneinander, je C₁-C₈-Alkyl, C₅-C₆-Cycloalkyl oder unsubstituiertes oder durch Halogen, Niederalkyl, Niederalkoxy oder Niederalkoxycarbonyl substituiertes Benzyl oder Phenyl oder die Substituentenpaare (R₅ und R₆) und (R₇ und R₈), unabhängig voneinander, je zusammen mit dem jeweiligen gemeinsamen Stickstoffatom Pyrrolidino, Piperidino oder Morpholino bedeuten und die Ringe B₁ und B₂ unsubstituiert oder durch Halogen, Niederalkyl, Niederalkoxycarbonyl oder Diniederalkylamino substituiert sind.

Unter den Bis-Phthalidlaktonverbindungen der Formel (2) sind diejenigen in denen R₅, R₆, R₇ und R₈ Niederalkyl oder Benzyl bedeuten und die Ringe B₁ und B₂ unsubstituiert oder durch Halogen, Niederalkyl oder Dimethylamino substituiert sind, besonders bevorzugt.

Von grossem Interesse sind Bis-Phthalidlaktonverbindungn der Formel 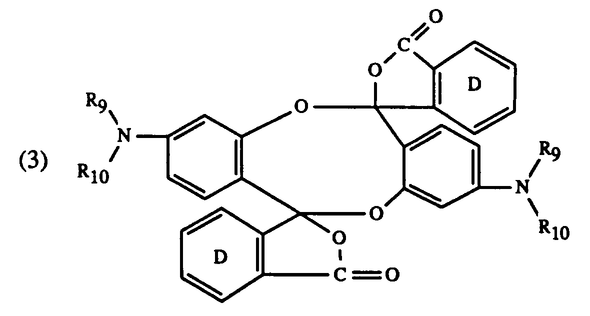
worin R₉ C₁-C₆-Alkyl, Cyclohexyl, Benzyl, Phenyl oder durch Methyl oder Chlor substituiertes Benzyl oder Phenyl und R₁₀ C₁-C₆-Alkyl, Benzyl oder durch Chlor oder Methyl substituiertes Benzyl bedeuten und die Ringe D unsubstituiert oder durch 1 bis 4 Chloratome substituiert sind.

Ganz besonders bevorzugt sind Bis-Phthalidlaktone der Formel (3), in der R₉ und R₁₀ C₁-C₄-Alkyl bedeuten und die Benzolringe D unsubstituiert sind.

Die erfindungsgemässen Bis-Phthalidlaktonverbindungen der Formeln (1) bis (3) werden dadurch hergestellt, dass man eine Ketosäureverbindung der Formel 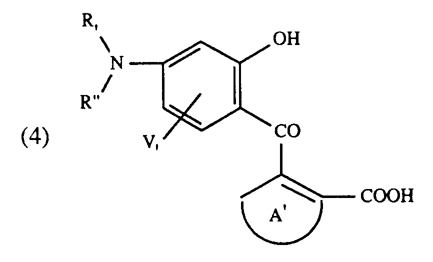
worin R', R'', V' und A' den Bedeutungen von R₁, R₂, R₃, R₄, V₁, V₂, A₁ und A₂ entsprechen, einzeln oder in Form eines Gemisches in Gegenwart eines Entwässerungsmittels, wie z.B. eines Säureanhydrids der Formel

(5) Z-CO-O-CO-Z'

worin Z und Z' gleich oder verschieden sein können und für Alkyl mit 1 bis 4 Kohlenstoffatomen oder Phenyl stehen, oder eines Säurehalogenids der Formel

(6) Z''-Q-Hal,

worin Z'' Halogen, Alkyl mit 1 bis 4 Kohlenstoffatomen oder Phenyl und Q -CO-, -SO-oder -SO₂- bedeuten und Hal für Halogen steht, reagieren lässt.

Die Anhydride der Formel (5) können als gemischte Anhydride angewandt werden, d.h. als Anhydride zweier verschiedener Säuren. Als Säurehalogenide der Formel (6) eignen sich beispielsweise Phosgen oder vor allem Thionylchlorid.

Die Reaktion erfolgt zweckmässigerweise in einem wasserfreien Medium, z.B. in einem Ueberschuss des verwendeten Anhydrids wie vor allem Essigsäureanhydrid oder in einem organischen Lösungsmittel, wie z.B. Benzol, Toluol, Xylol, Nitrobenzol, Chlorbenzol, Acetonitril, Propionitril oder Butyronitril oder besonders Aceton , Chloroform oder Methylethylketon.

Die Herstellung der Laktone erfolgt zweckmässigerweise bei einer Temperatur am oder unter dem Siedepunkt dieser Lösungsmittel, vorzugsweise bei einer Temperatur von 30 bis 110°C, insbesondere 30 bis 70°C.

Die Reaktionsdauer hängt von der Temperatur, dem Entwässerungsmittel und dem verwendeten Lösungsmittel ab und liegt in der Regel zwischen 1/2 und 5 Stunden, vorzugsweise 3/4 und 3 Stunden.

Die Isolierung des Endproduktes der Formel (1) erfolgt in allgemein bekannter Weise durch Abtrennen des gebildeten Niederschlages, nötigenfalls durch Zusatz von Wasser, Waschen und Trocknen oder durch Behandeln mit geeigneten organischen Lösungsmitteln, wie z.B. Methanol, Isopropanol, Aceton, Chloroform oder Toluol und gegebenenfalls Umkristallisieren des Produktes.

Die Bis-Phthalidlaktonverbindungen der Formeln (1) bis (3) sind normalerweise farblos oder höchstens schwach gefärbt. Wenn diese Verbindungen zusammen mit einer Kondensationskomponente und einem vorzugsweise sauren Entwickler, d.h. einem Elektronenakzeptor, in Kontakt gebracht werden, ergeben sie je nach der verwendeten Kondensationskomponente und dem Entwickler sofort intensive gelbe, orange, rote, violette, blaue, grüne, graue oder schwarze Farbtöne, die besonders licht- und sublimationsecht sind. Eine weitere Kombinationsmöglichkeit besteht darin, dass besagtes ternäres System zusammen mit einem oder mehreren konventionellen Farbbildnern z.B. 3,3-(Bis-aminophenyl-)-phthaliden wie CVL, 3-Indolyl-3-aminophenylaza- oder -diazaphthaliden, (3,3-Bis-indolyl-)-phthaliden, 3-Aminofluoranen, 6-Dialkylamino-2-dibenzylaminofluoranen, 6-Dialkylamino-3-methyl-2-arylaminofluoranen, 3,6-Bisalkoxyfluoranen, 3,6-Bis-diarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen verwendet wird.

In Kombination mit den Kondensationskomponenten, dem Entwickler und eventuell mit konventionellen Farbbildnern können erfindungsgemässe Bis-Phthalidlaktone sowohl in druckempfindlichen als auch in wärmeempfindlichen Aufzeichnungsmaterialien verwendet werden.

Als Kondensationskomponenten können alle in der Azochemie üblichen und aus der einschlägigen Literatur bekannten Kupplungskomponenten in Frage kommen, sofern sie keine sauren, wasserlöslich machenden Gruppen, wie z.B. Carboxyl- und Sulfonsäuregruppen enthalten.

Aus der Vielzahl der Möglichkeiten seien beispielsweise erwähnt: Kupplungskomponenten der Benzolreihe, der Naphthalinreihe, der offenkettigen methylenäktiven Verbindungen sowie der heterocyclischen Reihe.

Beispiele für die genannten Kupplungskomponenten sind N-substituierte Aminophenylethylenverbindungen, N-substituierte Aminophenylstyrolverbindungen, Acylacetarylamide, einwertige oder mehrwertige Phenole, Phenolether (Phenetole), 3-Aminophenolether, Aniline, Naphthylamine, Thionaphthene, Diarylamine, Naphthole, Naphtholcarbonsäureanilide, Morpholine, Pyrrolidine, Piperidine, Piperazine, Aminopyrimidine, Aminopyrazole, Pyrazolone, Thiophene, Acridine, Aminothiazole, Phenothiazine, Pyridone, Indole, Indolizine, Chinoline, Pyrimidone, Barbitursäuren, Carbazole, Benzomorpholine, Dihydrochinoline, Tetrahydrochinoline, Indoline, Kairoline oder Julolidine.

Besonders bevorzugte Kupplungskomponenten sind Aniline, wie Kresidine, Phenetidine oder N,N-Di-niederalkylaniline, 2-Niederalkylindole, 3-Niederalkylindole oder 2-Phenylindole, die jeweils durch C₁-C₈-Alkyl N-substituiert sein können sowie 5-Pyrazolone, wie z.B. 1-Phenyl-3-methyl-5-pyrazolon. Weitere bevorzugte Kupplungskomponenten sind 3-Niederalkyl-6-niederalkoxy- oder -6-diniederalkylaminoindole, die ebenfalls jeweils durch C₁-C₈-Alkyl N-substituiert sein können.

Spezifische Beispiele für Kupplungskomponenten sind 2-Amino-4-methoxytoluol, 3-Amino-4-methoxytoluol, 3-Amino-4-methoxy-1-ethylbenzol, 1,4-Dimethoxy-2-aminobenzol, N,N-Dimethylanilin, N,N-Diethylanilin, N,N-Di-benzylanilin, 3-Acetylamino-N,N-dipropylanilin, 3-n-Butoxy-N,N-Di-n-Butylanilin, 2-Methyl-5-acetyloxy-N,N-diethylanilin, 4-Ethoxy-diphenylamin, 3-Ethoxy-N,N-dimethylanilin, m-Phenetidin, 3-Ethoxy-N,N-diethylanilin, 1,3-Bis-dimethylaminobenzol, 3-Hydroxy-N,N-(di-2-cyclohexylethyl)aminobenzol, 1,1-(4'-Diethylamino- phenyl)ethylen, 1-Phenyl-3-methyl-5-pyrazolon, 1-Phenyl-5-methyl-3-pyrazolon, 1-(2'-Chlorphenyl)-5-methyl-3-pyrazolon, 2-Phenyl-4,6-bis-methylaminopyrimidin, N-Ethylcarbazol, N-Methylpyrrol, 2-Methylindol, 2-Phenylindol, 1,2-Dimethylindol, 1-Ethyl-2-methylindol, 1-n-Octyl-2-methyl-indol, 1-Methyl-2-phenylindol, 1-Ethyl-2-phenylindol, 3-Methyl-6-methoxyindol, 3-Methyl-6-dimethylaminoindol, 1-Ethyl-3-methyl-6-methoxyindol, 1-Ethyl-3-methyl-6-dimethylaminoindol, α-Naphthol, β-Naphthol, α-Naphthylamin, β-Naphthylamin, 3-Cyanoacetylaminophenol, Thionaphthen, Phenothiazin, 3-Methyl-5-amino-pyrazol, Pyrimidyl-2-essigsäureethylester, Iminodibenzyl, 1-Benzyl-2-methylindolin, 2,3,3-Trimethyl- indolenin, Benzthiazol-2-yl-acetonitril, 3-Phenyl-4-methyl- indolizin, 2,3-Diphenylindolizin.

Die Mengenverhältnisse, in denen Bis-Phthalidlaktone und Kondensationskomponenten verwendet werden, sind nicht kritisch, jedoch verwendet man sie vorzugsweise in stöchiometrischen Mengen.

Sowohl Bis-Phthalidlaktone als auch die Kondensationskomponenten können für sich allein oder als Mischungen in Form einer Kombination von zwei oder mehrerer derselben in dem Aufzeichnungsmaterial eingesetzt werden.

Als Entwickler können anorganische oder organische, für Aufzeichnungsmaterialien bekannte Farbentwickler, die fähig sind Elektronen anzuziehen (Elektronenakzeptoren), verwendet werden.

Typische Beispiele für anorganische Entwickler sind Aktivton-Substanzen, wie Attapulgus-Ton, Säureton, Bentonit, Montmorillonit; aktivierter Ton z.B. säureaktiviertes Bentonit oder Montmorillonit sowie Halloysit, Kaolin, Zeolith, Siliciumdioxid, Zirkondioxid, Aluminiumoxid, Aluminiumsulfat, Aluminiumphosphat oder Zinknitrat.

Bevorzugte anorganische Farbentwickler sind Lewis-Säuren, wie z.B. Aluminiumchlorid, Aluminiumbromid, Zinkchlorid, Eisen(III)chlorid, Zinntetrachlorid, Zinndichlorid, Zinntetrabromid, Titantetrachlorid Wismuttrichlorid, Tellurdichlorid oder Antimonpentachlorid.

Als organische Farbentwickler können feste Carbonsäuren, vorteilhafterweise aliphatische Dicarbonsäuren, wie z.B. Weinsäure, Oxalsäure, Maleinsäure, Zitronensäure, Citraconsäure oder Bernsteinsäure sowie Alkylphenolacetylenharz, Maleinsäure-Kolophonium-Harz, Carboxypolymethylen oder ein teilweise oder vollständig hydrolisiertes Polymerisat von Maleinsäureanhydrid mit Styrol, Ethylen oder Vinylmethylether verwendet werden.

Als organische Farbentwickler eignen sich insbesondere Verbindungen mit einer phenolischen Hydroxylgruppe. Diese können sowohl einwertige als auch mehrwertige Phenole sein. Diese Phenole können durch Halogenatome, Carboxylgruppen, Alkylreste, Aralkylreste, wie α-Methylbenzyl, α,α-Di-methylbenzyl, Arylreste, Acylreste, wie Arylsulfonyl, oder Alkoxycarbonylreste oder Aralkoxycarbonylreste, wie Benzyloxycarbonyl substituiert sein.

Spezielle Beispiele für als Entwickler geeignete Phenole sind 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethylester oder -benzylester, 2,4-Dihydroxybenzoesäuremethylester, 4-Hydroxydiphenylsulfon, 4'-Hydroxy-4-methyldiphenylsulfon, 4'-Hydroxy-4-isopropoxydiphenylsulfon, 4-Hydroxy-acetophenon, 2,4-Dihydroxybenzophenon , 2,2'-Dihydroxydiphenyl, 2,4-Dihydroxydiphenylsulfon, 4,4'-Cyclohexylidendiphenol, 4,4'-Isopropylidendiphenol, 4,4'-Isopropyliden-bis-(2-methylphenol), 4,4-Bis-(4-hydroxyphenyl)valeriansäure, 1-Phenyl-2,2-bis-(4-hydroxyphenyl)-butan, 1-Phenyl-1,1-bis-(4-hydroxyphenyl)-butan, Resorcin, Hydrochinon, Pyrogallol, Phloroglucin, p-, m-, o-Hydroxybenzoesäure, 3,5-Di-(α-methylbenzyl)-salicylsäure, 3,5-Di-(α,α-dimethylbenzyl)-salicylsäure, Salicylosalicylsäure, Gallussäurealkylester, Gallussäure, Hydroxyphthalsäure, Hydroxyphthalsäuredimethylester, 1-Hydroxy-2-naphthoesäure oder Phenol-Formaldehyde-Vorpolymerisate die auch mit Zink modifiziert sein können. Von den aufgezählten Carbonsäuren sind die Salicylsäurederivate bevorzugt, die vorzugsweise als Zinksalze eingesetzt werden. Besonders bevorzugte Zinksalicylate sind in EP-A-181 283 oder DE-A-2 242 250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2'-Hydroxyphenyl-)benztriazolen, Benzophenonen, Cyanoacrylaten, Salicylsäurephenylestern eingesetzt werden. Beispiele für Pigmente sind: Talk, Titandioxid, Aluminiumoxid, Aluminiumhydroxy, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Das Mischungsverhältnis des Entwicklers zu den anderen zwei Kompenenten hängt von der Art der drei Komponenten, der Art des Farbumschlags, von der Farbreaktionstemperatur und selbstverständlich auch von der gewünschten Farbkonzentration ab. Es werden zufriedenstellende Ergebnisse erzielt, wenn die farbentwickelnde Komponente in Mengen von 0,1, vorzugsweise mindestens 0,5 bis 100 Gew. Teilen pro Teil der Bis-Phthalidlaktone und Kondensationskomponenten zusammen eingesetzt werden.

Für das druckempfindliche Aufzeichnungsmaterial werden sowohl Bis-Phthalidiaktone als auch Kondensationskomponenten vorzugsweise gemeinsam oder auch getrennt in einem organischen Lösungsmittel gelöst und die erhaltenen Lösungen werden zweckmässigerweise eingekapselt nach Verfahren, wie z.B. in den U.S. Patentschriften 2 712 507, 2 800 457, 3 016 308, 3 429 827, 3 578 605 und 4 100 103 oder in den britischen Patentschriften 989 264, 1 156 725, 1 301 052 oder 1 355 124 beschrieben werden. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid, Polyharnstoff oder Polyurethan. In manchen Fällen genügt, dass lediglich das Bis-Phthalidlakton eingekapselt wird. Die Einkapselung ist in der Regel erforderlich um die farbbildenden Komponenten vom Entwickler zu trennen und somit eine frühzeitige Reaktion zu verhindern. Letzteres kann auch erzielt werden, indem man die farbbildenden Komponenten in schaum-, schwamm- oder bienenwabenartigen Strukturen einarbeitet.

Beispiele für geeignete Lösungsmittel sind vorzugsweise nichtflüchtige Lösungsmittel z.B. halogeniertes Benzol, Diphenyle oder Paraffin, wie z.B. Chlorparaffin, Trichlorbenzol, Monochlordiphenyl, Dichlordiphenyl, oder Trichlordiphenyl; Ester, wie z.B. Dibutyladipat, Dibutylphthalat, Dioctylphthalat, Butylbenzyladipat, Trichlorethylphosphat, Trioctylphosphat, Tricresylphosphat; aromatische Ether wie Benzylphenylether; Kohlenwasserstofföle, wie Parrafinöl oder Kerosin, aromatische Kohlenwasserstoffe z.B. mit Isopropyl, Isobutyl, sek.-Butyl oder tert.-Butyl alkylierte Derivate von Diphenyl, Nahthalin oder Terphenyl, Dibenzyltoluol, partiell hydriertes Terphenyl, mono- bis tetraC₁-C₃-alkylierte Diphenylalkane, Dodecylbenzol, benzylierte Xylole, Phenylxylylethan, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die farbbildende Komponenten enthaltenden Mikrokapseln können zur Herstellung von druckempfindlichen Kopiermaterialien der verschiedensten bekannten Arten verwendet werden. Die verschiedenen Systeme unterscheiden sich im wesentlichen durch die Anordnung der Kapseln, der Farbreaktanten und durch das Trägermaterial voneinander.

Vorteilhaft ist eine Anordnung, bei der eingekapselte Komponenten in Form einer Schicht auf der Rückseite eines Uebertragungsblattes und der Entwickler (Elektronenakzeptor) in Form einer Schicht auf der Vorderseite eines Empfangsblattes vorhanden sind. Eine andere Anordnung der Bestandteile besteht darin, dass die farbbildende Komponenten enthaltenden Mikrokapseln und der Entwickler in oder auf dem gleichen Blatt in Form einer oder mehrerer Einzelschichten vorliegt oder im Trägermaterial eingebaut ist.

Zur Erzielung der gewünschten Farbe, kann die Kapselmasse, welche die farbbildenden Komponenten enthält, mit weiteren Kapseln, welche konventionelle Farbbildner enthalten, vermischt werden. Aehnliche Resultate werden erzielt, wenn man die farbbildenden Kondensationskomponenten gemeinsam mit einem oder mehreren der konventionellen Farbbildner enkapsuliert.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie z.B. Gummiarabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunstfolie sein.

Vorzugsweise besteht das Durchschreibematerial auch darin, dass es eine kapselfreie, farbbildende Komponenten enthaltende Schicht und eine farbentwickelnde Schicht, die als Farbentwickler mindestens ein anorganisches Metallsalz vor allem Halogenide oder Nitrate eines mehrwertigen Metalles, wie z.B. Zinkchlorid, Zinnchlorid, Zinknitrat oder deren Gemische enthält, aufweist.

Das erfindungsgemässe ternäre Farbbildungssystem eignet sich insbesondere zur Herstellung eines wärmeempfindlichen Aufzeichnungsmaterials für die Thermographie, wobei die drei Komponenten beim Erhitzen miteinander unter Farbbildung in Berührung kommen und Aufzeichnungen auf dem Trägermaterial hinterlassen.

Das wärmeempfindliche Aufzeichnungsmaterial enthält in der Regel mindestens einen Schichtträger, die drei Komponenten und gegebenenfalls auch ein Bindemittel und/oder Wachs. Gewünschtenfalls können zusätzlich auch Aktivatoren z.B. Benzyldiphenyl, Benzyloxy-naphthalin oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechenmaschinen, Druckern, Faksimile- oder Kopiermaschinen oder in medizinischen und technischen Aufzeichnungsgeräten und Messinstrumenten, wie z.B. Elektrocardiographen, oder zur Beschriftung von Etiketten oder z.B. Fahrkarten (Zug- oder Flugbillete) verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass die farbbildenden Komponenten in einer Bindemittelschicht gelöst oder dispergiert sind und in einer zweiten Schicht der Entwickler in dem Bindemittel gelöst und dispergiert ist. Eine andere Möglichkeit besteht darin, dass alle drei Komponenten in derselben Schicht dispergiert sind. Die Schicht bzw. Schichten werden in spezifischen Bezirken mittels Wärme erweicht oder verschmolzen; dann kommen an diesen Punkten, an denen Wärme angewendet wird, die drei Komponenten untereinander in Kontakt, und es entwickelt sich sofort die erwünschte Farbe.

Vorzugsweise werden zur Herstellung des wärmeempfindlichen Aufzeichnungsmaterials schmelzbare, filmbildende Bindemittel verwendet. Diese Bindemittel sind normalerweise wasserlöslich, während die drei Komponenten in Wasser unlöslich sind. Das Bindemittel sollte in der Lage sein, die drei Komponenten bei Raumtemperatur zu dispergieren und auf dem Schichtträger zu fixieren.

Wasserlösliche oder mindestens in Wasser quellbare Bindemittel sind z.B. hydrophile Polymerisate, wie Polivinylalkohol, Alkalimetall-Polyacrylate, Hydroxyethylcellulose, Methylcellulose, Carboxymethylcellulose, Polyacrylamid, Polyvinylpyrrolidon, carboxylierte Butadien-Styrolco- polymerisate, Gelatine, Stärke oder veresterte Maisstärke.

Wenn die drei Komponenten in zwei oder drei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Polystyrol, Styrol/Butadien-Mischpolymerisate, Polymethylacrylate, Ethylcellulose, Nitrocellulose und Polyvinylcarbazol verwendet werden. Die bevorzugte Anordnung ist jedoch diejenige, bei der alle drei Komponenten in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Spezielle Beispiele für wasserlösliche Polymermaterialien sind Polyvinylalkohol, Stärke, Stärkederivate, Cellulosederivate, wie Methoxycellulose, Hydroxyethylcellulose, Carboxymethylcellulose, Methylcellulose oder Ethylcellulose, Natriumpolyacrylat, Polyvinylpyrrolidon, Polyacrylamid/ Acrylsäureester-Copolymere, Acrylamid/Acrylsäureester/Methacrylsäure-Copolymere, Styrol/Maleinsäureanhydrid-Copolymer-Alkalisalze, Isobuten/ Maleinsäureanhydrid-Copolymer-Alkalisalze, Polyacrylamid, Natriumalginat, Gelatine, Casein, wasserlösliche Polyester oder Carboxyl-modifizierter Polyvinylalkohol.

Gegebenenfalls können in der Schutzschicht in Kombination mit den genannten wasserlöslichen Polymerharzen z.B. die folgenden wasserunlöslichen Harze angewandt werden: Polyvinylacetat, Polyurethane, Styrol/Butadien-Copolymere, Polyacrylsäure, Polyacrylsäureester, Vinylchlorid/Vinylacetat-Copolymere, Vinylalkohol-Vinylacetat-Maleinsäure-Terpolymere, Polybutylmethacrylat, Ethylen/Vinylacetat-Copolymere und Styrol/Butadien/Acrylderivat-Copolymere.

Sowohl die thermoreaktiven Schichten als auch die Harzschichten können weitere Zusätze enthalten. Zur Verbesserung des Weissgrades oder der Thermokopfeignung des Aufzeichnungsmaterials und zur Verhinderung des Festklebens der erhitzten Feder oder Platte können diese Schichten, z.B. Antioxidantien, UV-Absorber, Lösungshilfen, Talk, Titandioxyd, Zinkoxyd, Aluminiumoxyd, Aluminiumhydroxyd, Calciumcarbonat (z.B. Kreide), Tone oder auch organische Pigmente, wie z.B. Harnstoff-Formaldehydpolymerisate, enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-stearoylethylendiamid, Stearinsäureamid, Phthalsäureanhydrid, Benzyloxybenzoesäurebenzylester, Metallstearate, wie z.B. Zinkstearat, Phthalsäurenitril, Dibenzylterephthalat, Dimethylterephthalat oder andere entsprechende, schmelzbare Produkte, welche das gleichzeitige Schmelzen der Farbbildnerkomponenten und des Entwicklers induzieren, zugesetzt werden.

Bevorzugt enthalten thermographische Aufzeichnungsmaterialien Wachse, z.B. Carnaubawachs, Montanwachs, Paraffinwachs, Mikrowachs, Polyethylenwachs, Kondensate höherer Fettsäureamide und Formaldehyd oder Kondensate höherer Fettsäuren und Ethylendiamin.

Zur Verbesserung der Verwendbarkeit der thermochromatischen Materialien können die drei Komponenten in Mikrokapseln eingeschlossen sein. Dazu können beliebige, obengenannte an sich bekannte Verfahren zum Einschliessen von Farbbildnern oder anderen Wirkstoffen in Mikrokapseln verwendet werden.

In den folgenden Herstellungsvorschriften und Beispielen beziehen sich die angegebenen Prozentsätze, wenn nichts anderes angegeben ist, auf das Gewicht. Teile sind Gewichtsteile.

Beispiel 1: 15,6 g 4-Diethylamino-2-hydroxybenzophenon-2'-carbonsäure werden in 100 ml Aceton und 30 ml Essigsäureanhydrid bei 20-25°C angeschlämmt und auf 60°C erwärmt. Man rührt während 1 Stunde bei 55-60°C und lässt anschliessend abkühlen. Man trägt die Reaktionsmasse auf 70 ml Wasser aus, wobei die Temperatur auf 50°C steigt. Nach Abkühlen auf 20°C rührt man 1 Stunde und saugt den Niederschlag ab. Nach dem Trocknen erhält man 5,7 g einer Verbindung der Formel 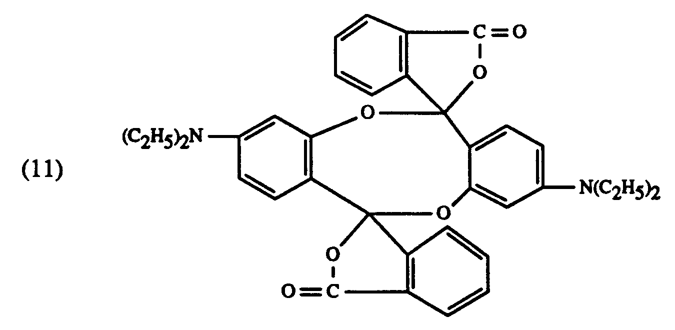
Nach Umkristallisieren aus Methylethylketon-Petrolether weist die Verbindung einen Schmelzpunkt von 206-207°C auf.

Beispiel 2: 12,5 g 4-Diethylamino-2-hydroxybenzophenon-2'-carbonsäure werden in 80 ml Chloroform eingerührt. Man lässt innert 10 Minuten 5,1 ml Thionylchlorid zutropfen, wobei die Temperatur von 25 auf 36°C steigt. Man erwärmt langsam bis 50°C, gibt weitere 20 ml Chloroform zu und hält 2 1/2 Stunden bei 50°C. Hierauf gibt man nochmals 2,5 ml Thionylchlorid zu und hält eine weitere 1/2 Stunde bei 50°C. Die Suspension wird bei 20°C filtriert und mit Chloroform und Wasser gewaschen. Nach dem Trocknen erhält man 11,7 g der Verbindung der Formel (11). Nach Umkristallisation aus Toluol weist die Verbindung einen Schmelzpunkt von 206-207°C auf.

Beispiel 3: 11,2 g 4-Diethylamino-2-hydroxy-4'-tert.butyl-benzophenon-2'-carbonsäure werden in 60 ml Chloroform eingerührt. Man lässt 3,8 ml Thionylchlorid zutropfen, wobei die Temperatur auf 35°C steigt. Man erwärmt auf 50°C und hält 3 Stunden bei 50°C, worauf die Gasentwicklung praktisch beendet ist. Die Lösung wird zur Trockene eingedampft und der Rückstand mit Aceton behandelt. Die erhaltenen Kristalle werden abfiltriert, mit Aceton gewaschen und getrocknet. Man erhält 3 g einer Verbindung der Formel 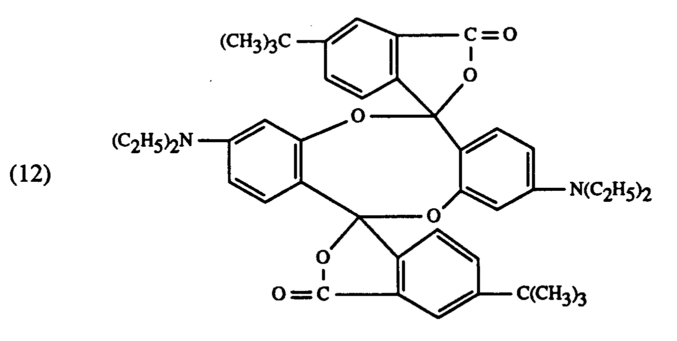
Der Schmelzpunkt ist 178-179°C.

Beispiel 4: 14,8 g 4-Di-n-butylamino-2-hydroxybenzophenon-2'-carbonsäure werden in 80 ml Aceton suspendiert. Die Suspension wird mit 24 ml Acetanhydrid versetzt und zum Rückfluss erhitzt. Man rührt während 1 1/2 Stunden und lässt anschliessend abkühlen. Man fügt 70 ml Wasser hinzu und lässt über Nacht ausrühren. Der Niederschlag wird abfiltriert, mit Wasser gewaschen und bei 70-80°C getrocknet. Man erhält 13,3 g der Verbindung der Formel 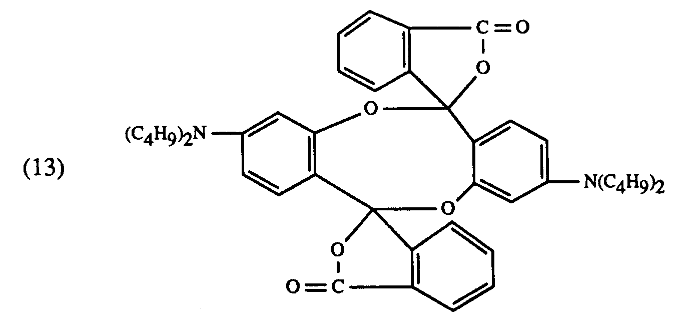
Der Schmelzpunkt ist 150-154°C.

Beispiel 5: Zur Herstellung einer Dispersion A werden 6 g des Zinksalicylates gemäss EP-A-181283, Beispiel 1, 21 g einer 10%igen wässrigen Lösung von Polyvinylalkohol (Polyviol V03/140) und 12 g Wasser mit Glaskugeln bis zu einer Korngrösse von 2-4 »m gemahlen.

Zur Herstellung einer Dispersion B werden 0,87 g der Verbindung der Formel (11) gemäss Beispiel 1,5 g einer 10%igen wässrigen Lösung von Polyvinylalkohol (Polyviol V03/140) und 2,9 g Wasser mit Glaskugeln bis zu einer Korngrösse von 2-4 »m gemahlen.

Zur Herstellung einer Dispersion C werden 0,57 g 2-Phenylindol, 2 g einer 10%igen wässrigen Lösung von Polyvinylalkohol (Polyviol V03/140) und 1,1 g Wasser bis zu einer Korngrösse von 2-4 »m gemahlen.

Anschliessend werden die Dispersionen A, B und C vermischt und mit einem Rakel so auf ein Papier mit einem Flächengewicht von 50 g/m² aufgetragen, dass das aufgebrachte Material 4 g/m² Trockengewicht entspricht. Durch Verwendung des Papiers in einem Faksimile-Gerät (Infotec 6510) entwickelt sich eine lichtechte, schwarze Farbe.

Beispiel 6: 0,15 g der Verbindung Formel (11) werden in 10 g Diisopropylnaphthalin gelöst 0,1 g Aminohydrochinon-dimethylether(1,4-Dimethoxy-2-aminobenzol) werden ebenfalls in 10 g Diisopropylnaphthalin gelöst und dann die beiden Lösungen bei 25°C vermischt. Die erhaltene Mischung wird mit einer Druckwalze auf ein Blatt Papier appliziert, welches Zink-2,5-bis-α-methylbenzylsalicylat enthält. Anschliessend wird das Papier während 1 Minute bei 150°C wärmebehandelt, wobei sich eine intensive und lichtechte gelbe Farbe mit λ max. 420 nm entwickelt.

Beispiel 7: Ersetzt man in Beispiel 6 Aminohydrochinon-dimethylether durch 0,1 g 2-Phenyl-4,6-bis-N-methylamino-pyrimidin und verfährt ansonst wie dort beschrieben, resultiert nach der Wärmebehandlung eine rote Farbe, λ max. 580 nm.

Beispiel 8: Ersetzt man in Beispiel 6 Aminohydrochinon-dimethylether durch 0,1 g 2-Phenylindol und verfährt sonst wie dort beschrieben, erhält man nach der Wärmebehandlung eine blaue Farbe, λ max. 600 nm.

Beispiel 9: Zur Mischung der Dispersionen A, B und C aus Beispiel 5 mischt noch eine Dispersion D, hergestellt aus 1 g 2-Phenylamino-3-methyl-6-diethylaminofluoran, 3,5 g einer 10%igen wässrigen Lösung von Polyvinylalkohol(Polyviol V03/140) und 2 g Wasser. Die vereinigte Mischung wird mit einem Rakel so auf ein Papier aufgetragen, dass das aufgebrachte Material nach dem Trocknen 2,3 g/m² beträgt. Unter Verwendung des Papiers im Faksimile-Gerät (Infotec 6510) erhält man eine grau-schwarze Kopie mit guter Lichtechtheit.

## Patentansprüche

1. Bis-Phthalidlaktonverbindungen der Formel 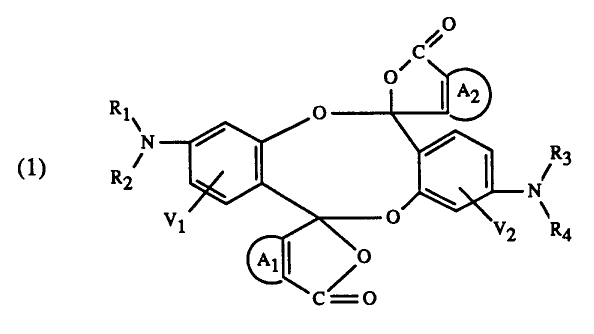 worin R₁, R₂, R₃ und R₄, unabhängig voneinander, je Wasserstoff, unsubstituiertes oder durch Halogen, Hydroxy, Cyano, Tetrahydrofuryl oder Niederalkoxy substituiertes Alkyl mit höchstens 12 Kohlenstoffatomen, Acyl mit 1 bis 12 Kohlenstoffatomen, Cycloalkyl mit 5 bis 10 Kohlenstoffatomen oder unsubstituiertes oder durch Halogen, Cyano, Nitro, Trifluormethyl, Niederalkyl, Niederalkoxy, Niederalkoxycarbonyl, -NX'X'' oder 4-NX'X''-phenylamino substituiertes Aralkyl oder Aryl, worin X' und X'', unabhängig voneinander, Wasserstoff, Niederalkyl, Cyclohexyl, Benzyl oder Phenyl darstellen, oder die Substituentenpaare (R₁ und R₂) und (R₃ und R₄) je zusammen mit dem gemeinsamen Stickstoffatom einen fünf- oder sechsgliedrigen heterocyclischen Rest, V₁ und V₂ Wasserstoff, Halogen, Niederalkyl, C₁-C₁₂-Alkoxy, C₁-C₁₂-Acyloxy, Benzyl, Phenyl, Benzyloxy, Phenyloxy, durch Halogen, Cyano, Niederalkyl oder Niederalkoxy substituiertes Benzyl oder Benzyloxy, oder die Gruppe -NT₁T₂, T₁ und T₂, unabhängig voneinander, je Wasserstoff, Niederalkyl, C₅-C₁₀-Cycloalkyl, unsubstituiertes oder durch Halogen, Cyano, Niederalkyl oder Niederalkoxy substituiertes Benzyl, oder Acyl mit 1 bis 8 Kohlenstoffatomen und T₁ auch unsubstituiertes oder durch Halogen, Cyano, Niederalkyl oder Niederalkoxy substituiertes Phenyl, bedeuten und die Ringe A₁ und A₂, unabhängig voneinander, einen aromatischen oder heterocyclischen Rest mit 6 Ringatomen darstellen, die einen aromatischen annellierten Ring aufweisen können, wobei sowohl die Ringe A₁ und A₂ als auch die annellierten Ringe substituiert sein können.

2. Bis-Phthalidlaktonverbindungen gemäss Anspruch 1, dadurch gekennzeichnet, dass in Formel (1) -NR₁R₂ und -NR₃R₄ identisch sind.

3. Bis-Phthalidlaktonverbindungen gemäss einem der Ansprüche 1 und 2, dadurch gekennzeichnet, dass in Formel (1) R₁, R₂, -R₃, R₄ unabhängig voneinander, Niederalkyl, Cyano-Niederalkyl, Cyclohexyl, Tolyl oder Benzyl bedeuten oder -NR₁R₂ oder -NR₃R₄ Pyrrolidino, N-Niederalkyl-N-tetrahydrofurfurylamino, 4-Diniederalkylaminophenylamino oder 4-(4'-Phenylamino-phenylamino)-phenylamino darstellen.

4. Bis-Phthalidlaktonverbindungen gemäss einem der Ansprüche 1 bis 3, dadurch gekennzeichnet, dass in Formel (1) V₁ und V₂ Wasserstoff, Halogen, Niederalkyl, Niederalkoxy oder -NT₁T₂ bedeuten und von T₁ und T₂ eines Niederalkyl oder C₁-C₈-Acyl und das andere Wasserstoff oder Niederalkyl ist.

5. Bis-Phthalidlaktonverbindungen gemäss einem der Ansprüche 1 bis 4, dadurch gekennzeichnet, dass in Formel (1) die Ringe A₁ und A₂ einen gegebenenfalls substituierten Benzol-, Naphthalin-, Pyridin-, Pyrazin-, Chinoxalin- oder Chinolinring darstellen.

6. Bis-Phthalidlaktonverbindungen gemäss Anspruch 1, dadurch gekennzeichnet, dass sie der Formel 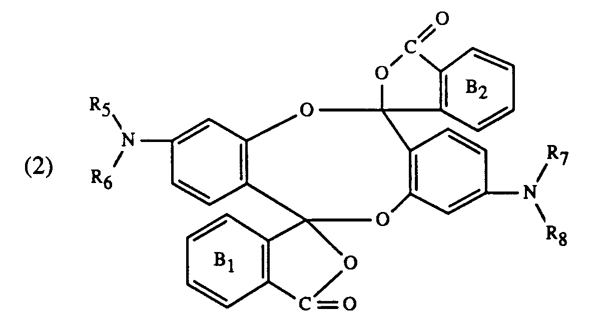 entsprechen, worin R₅, R₆, R₇ und R₈, unabhängig voneinander, je C₁-C₈-Alkyl, C₅-C₆-Cycloalkyl oder unsubstituiertes oder durch Halogen, Niederalkyl, Niederalkoxy oder Niederalkoxycarbonyl substituiertes Benzyl oder Phenyl oder die Substituentenpaare (R₅ und R₆) und (R₇ und R₈), unabhängig voneinander, je zusammen mit dem jeweiligen gemeinsamen Stickstoffatom Pyrrolidino, Piperidino oder Morpholino bedeuten und die Ringe B₁ und B₂ unsubstituiert oder durch Halogen, Niederalkyl, Niederalkoxycarbonyl oder Diniederalkylamino substituiert sind.

7. Bis-Phthalidlaktonverbindungen gemäss Anspruch 6, dadurch gekennzeichnet, dass in Formel (2) R₅, R₆, R₇ und R₈ Niederalkyl oder Benzyl bedeuten und die Ringe B₁ und B₂ unsubstituiert oder durch Halogen, Niederalkyl oder Dimethylamino substituiert sind.

8. Bis-Phthalidlaktonverbindungen gemäss Anspruch 1, dadurch gekennzeichnet, dass sie der Formel 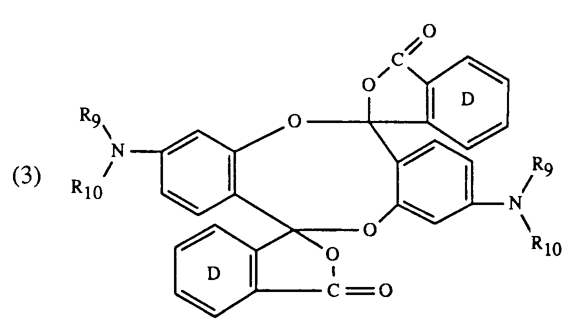 entsprechen, worin R₉ C₁-C₆-Alkyl, Cyclohexyl, Benzyl, Phenyl oder durch Methyl oder Chlor substituiertes Benzyl oder Phenyl und R₁₀ C₁-C₆-Alkyl, Benzyl oder durch Chlor oder Methyl substituiertes Benzyl bedeuten und die Ringe D unsubstituiert oder durch 1 bis 4 Chloratome substituiert sind.

9. Bis-Phthalidlaktonverbindungen gemäss Anspruch 8, dadurch gekennzeichnet, dass in Formel (3) R₉ und R₁₀ C₁-C₄-Alkyl bedeuten und die Benzolringe D unsubstituiert sind.

10. Verfahren zur Herstellung von Bis-Phthalidlaktonverbindungen gemäss Anspruch 1, dadurch gekennzeichnet, dass man eine Ketosäure der Formel 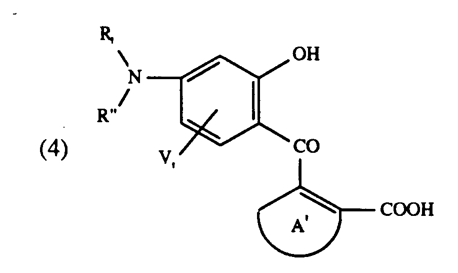 worin R', R'', V' und A' den Bedeutungen von R₁, R₂, R₃, R₄, V₁, V₂, A₁ und A₂ gemäss Anspruch 1 entsprechen, einzeln oder in Form eines Gemisches in Gegenwart eines Entwässerungsmittels reagieren lässt.

11. Verwendung von Bis-Phthalidlaktonverbindungen gemäss einem der Ansprüche 1 bis 9 als Farbbildungskomponenten in einem druckempfindlichen oder wärmeempfindlichen Aufzeichnungsmaterial.

12. Druck- oder wärmeempfindliches Aufzeichnungsmaterial dadurch gekennzeichnet, dass es in seinem Farbreaktantensystem (A) eine Bis-Phthalidlaktonverbindung gemäss einem der Ansprüche 1 bis 9, (B) eine organische Kondensationskomponente und (C) eine elektronenanziehende und farbentwickelnde Komponente enthält.

13. Aufzeichnungsmaterial gemäss Anspruch 12, dadurch gekennzeichnet, dass die Kondensationskomponente (B) eine N-substituierte Aminophenylethylen-, N-substituierte Aminophenylstyrol-, Acylacetarylamid-, einwertige oder mehrwertige Phenol-, Phenolether-, 3-Aminophenolether-, Anilin-, Naphthylamin-, Diarylamin-, Naphthol-, Naphtholcarbonsäureanilid-, Aminopyrimidin-, Aminopyrazol-, Pyrazolon-, Thiophen-, Thionaphthen-, Phenothiazin-, Aminothiazol-, Acridin-, Pyridon-, Indol-, Carbazol-, Kairolin-, Indolizin-, Julolidin-, Morpholin-, Pyrrolidin-, Piperidin-, Piperazin-, Indolin-, Chinolin-, Pyrimidon-, Barbitursäure-, Benzomorpholin-, Dihydrochinolin- oder Tetrahydrochinolinverbindung ist.

14. Aufzeichnungsmaterial gemäss einem der Ansprüche 12 und 13, dadurch gekennzeichnet, dass die Kondensationskomponente (B) eine 5-Pyrazolonverbindung, eine Kresidin-, Phenetidin- oder N,N-Diniederalkylanilinverbindung, eine 3-Niederalkyl-6-dinierderalkylaminoindolverbindung, 2-Niederalkylindol, 2-Phenylindol, eine 3-Niederalky-6-niederalkoxyindolverbindung oder eine durch C₁-C₈-Alkyl N-substituierte 2-Niederalkylindol-, 2-Phenylindo-, 3-Niederalkyl-6-niederalkoxyindol- oder 3-Niederalkyl-6-dinierderalkylaminoindolverbindung ist.

15. Aufzeichnungsmaterial gemäss einem der Ansprüche 12 bis 14, dadurch gekennzeichnet, dass die farbentwickelnde Komponente (C) eine Lewis-Säure, ein Sauerton, eine feste Carbonsäure oder eine Verbindung mit einer phenolischen Hydroxylgruppe ist.

16. Aufzeichnungsmaterial gemäss einem der Ansprüche 12 bis 15, dadurch gekennzeichnet, dass es druckempfindlich ist.

17. Aufzeichnungsmaterial gemäss Anspruch 16, dadurch gekennzeichnet, dass Komponenten (A) und (B) in einem organischen Lösungsmittel gelöst sind.

18. Aufzeichnungsmaterial gemäss Anspruch 17, dadurch gekennzeichnet, dass Komponenten (A) und (B) in Mikrokapseln eingekapselt sind.

19. Aufzeichnungsmaterial gemäss einem der Ansprüche 16 bis 18, dadurch gekennzeichnet, dass Komponenten (A) und (B) in Form einer oder zwei Schichten auf der Rückseite eines Uebertragungsblattes und Komponente (C) in Form einer Schicht auf der Vorderseite eines Empfangsblattes vorhanden sind.

20. Aufzeichnungsmaterial gemäss einem der Ansprüche 12 bis 15, dadurch gekennzeichnet, dass es wärmeempfindlich ist.

21. Aufzeichnungsmaterial gemäss Anspruch 20, dadurch gekennzeichnet, dass es 1 bis 4 Schichtträger aufweist, in denen Komponenten (A), (B) und (C) und gegebenenfalls ein Bindemittel und/oder Wachs vorhanden sind.

22. Aufzeichnungsmaterial gemäss einem der Ansprüche 20 und 21, d.g. dass es zusätzlich einen Aktivator enthält.

23. Aufzeichnungsmaterial gemäss einem der Ansprüche 12 bis 22, dadurch gekennzeichnet, dass die farbbildenden Komponenten (A) und (B) mit einem oder mehreren konventionellen Farbbildnern vorhanden sind.

24. Aufzeichnungsmaterial gemäss Anspruch 23, dadurch gekennzeichnet, dass als konventionelle Farbbildner 3,3-(Bis-aminophenyl-)-phthalide, 3-Indolyl-3-aminophenylaza- oder -diazaphthalide, (3,3-Bis-indolyl-)-phthalide, 3-Aminofluorane, 6-Dialkylamino-2-benzylaminofluorane, 6-Dialkylamino-3-methyl-2-arylaminofluorane, 3,6-Bisalkoxyfluorane, 3,6,-Bis-diarylaminofluorane, Leukoauramine, Spiropyrane, Spirodipyrane, Chromenopyrazole, Chromenoindole, Phenoxazine, Phenothiazine, Chinazoline, Rhodaminlaktame, Carbazolylmethane oder weiteren Triarylmethyl-leukofarbstoffe vorhanden sind.

## Claims

1. A bisphthalide lactone compound of formula 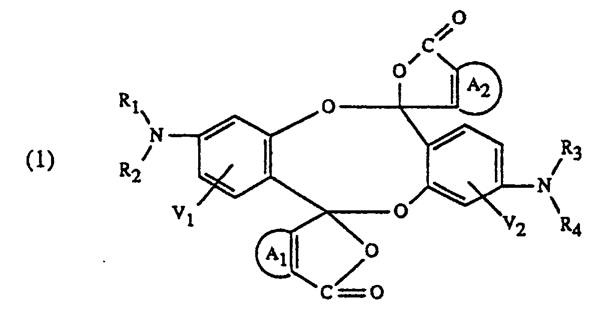 wherein R₁, R₂, R₃ and R₄ are each independently of one another hydrogen, alkyl of at most 12 carbon atoms which is unsubstituted or substituted by halogen, hydroxyl, cyano, tetrahydrofuryl or lower alkoxy, or are acyl of 1 to 12 carbon atoms, cycloalkyl of 5 to 10 carbon atoms, aralkyl or aryl, or aralkyl or aryl each substituted by halogen, cyano, nitro, trifluoromethyl, lower alkyl, lower alkoxy, lower alkoxycarbonyl, -NX'X'' - or 4-NX'X'' -phenylamino, wherein X' and X'' are each independently of the other hydrogen, lower alkyl, cyclohexyl, benzyl or phenyl, or the pairs of substituents (R₁ and R₂) and (R₃ and R₄) are each, together with their common nitrogen atom, a five- or six-membered heterocyclic radical, V₁ and V₂ are hydrogen, halogen, lower alkyl, C₁-C₁₂alkoxy, C₁-C₁₂acyloxy, benzyl, phenyl, benzyloxy, phenyloxy, or benzyl or benzyloxy, each substituted by halogen, cyano, lower alkyl or lower alkoxy, or are the group -NT₁T₂, in which T₁ and T₂ are each independently of the other hydrogen, lower alkyl, C₅-C₁₀cycloalkyl, benzyl or benzyl which is substituted by halogen, cyano, lower alkyl or lower alkoxy, or are acyl of 1 to 8 carbon atoms, and T₁ is also phenyl or phenyl which is substituted by halogen, cyano, lower alkyl or lower alkoxy, and the rings A₁ and A₂ are each independently of the other an aromatic or heterocyclic radical of 6 ring atoms which may have an aromatic fused ring, which rings A₁ and A₂ as well as the fused rings may be substituted.

2. A bisphthalide lactone compound according to claim 1, wherein in formula (1) -NR₁R₂ and -NR₃R₄ are identical.

3. A bisphthalide lactone compound according to one of claims 1 and 2, wherein in formula (1) R₁, R₂, R₃ and R₄ are each independently of one another lower alkyl, cyano-lower alkyl, cyclohexyl, tolyl or benzyl, or -NR₁R₂ or -NR₃R₄ are pyrrolidino, N-lower alkyl-N-tetrahydrofurfurylamino, 4-di-lower alklaminophenylamino or 4-(4'-phenylaminophenylamino)phenylamino.

4. A bisphthalide lactone compound according to one of claims 1 to 3, wherein in formula (1) V₁ and V₂ are hydrogen, halogen, lower alkyl, lower alkoxy or -NT₁T₂, and one of T₁ and T₂ is lower alkyl or C₁-C₈acyl and the other is hydrogen or lower alkyl.

5. A bisphthalide lactone compound according to one of claims 1 to 4, wherein in formula (1) the rings A₁ and A₂ are an unsubstituted or a substituted benzene, naphthalene, pyridine, pyrazine, quinoxaline or quinoline ring.

6. A bisphthalide lactone compound according to claim 1 of formula 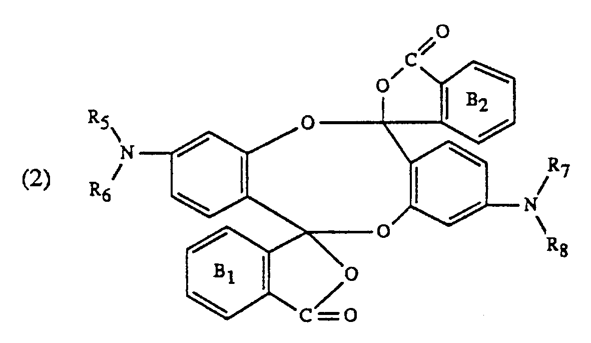 wherein R₅, R₆, R₇ and R₈ are each independently of one another C₁-C₈alkyl, C₅-C₆cycloalkyl, benzyl or phenyl, or benzyl or phenyl each substituted by halogen, lower alkyl, lower alkoxy or lower alkoxycarbonyl, or the pairs of substituents (R₅ and R₆) and (R₇ and R₈) are each independently of the other and together with their common nitrogen atom pyrrolidino, piperidino or morpholino, and the rings B₁ and B₂ are unsubstituted or substituted by halogen, lower alkyl, lower alkoxycarbonyl or di-lower alkylamino.

7. A bisphthalide lactone compound according to claim 6, wherein in formula (2) R₅, R₆, R₇ and R₈ are lower alkyl or benzyl, and the rings B₁ and B₂ are unsubstituted or substituted by halogen, lower alkyl or dimethylamino.

8. A bisphthalide lactone compound according to claim 1 of formula 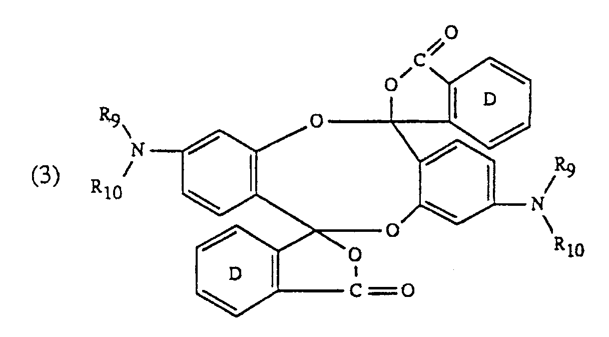 wherein R₉ is C₁-C₆alkyl, cyclohexyl, benzyl, phenyl, or methyl- or chloro-substituted benzyl or phenyl, and R₁₀ is C₁-C₆alkyl, benzyl, or chloro- or methyl-substituted benzyl, and the rings D are unsubstituted or substituted by 1 to 4 chlorine atoms.

9. A bisphthalide lactone compound according to claim 8, wherein in formula (3) R₉ and R₁₀ are C₁-C₄alkyl and the benzene rings D are unsubstituted.

10. A process for the preparation of a bisphthalide lactone compound according to claim 1, which comprises reacting a keto acid of formula 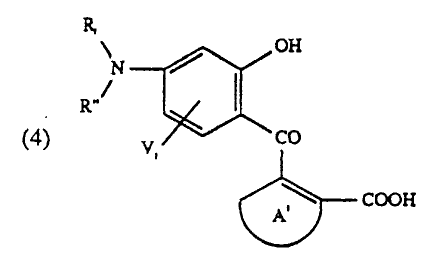 wherein R', R'', V' and A' have the meanings of R₁, R₂, R₃, R₄, V₁, V₂, A₁ and A₂ as defined in claim 1, individually or in the form of a mixture, in the presence of a dehydrating agent.

11. The use of a bisphthalide lactone compound according to one of claims 1 to 9 as a colour-forming component in a pressure-sensitive or heat-sensitive recording material.

12. A pressure-sensitive or heat-sensitive recording material which contains in its colour reactant system (A) a bisphthalide lactone compound according to one of claims 1 to 9, (B) an organic condensation component, and (C) an electron-attracting and colour-developing component.

13. A recording material according to claim 12, wherein the condensation component (B) is an N-substituted aminophenylethylene, N-substituted aminophenylstyrene, acylacetarylamide, monohydric or polyhydric phenol, phenol ether, 3-aminophenol ether, aniline, naphthylamine, diarylamine, naphthol, naphtholcarboxylic anilide, aminopyrimidine, aminopyrazole, pyrazolone, thiophene, thionaphthene, phenothiazine, aminothiazole, acridine, pyridone, indole, carbazole, kairoline, indolizine, julolidine, morpholine, pyrrolidine, piperidine, piperazine, indoline, quinoline, pyrimidone, barbituric acid, benzomorpholine, dihydroquinoline or tetrahydroquinoline compound.

14. A recording material according to one of claims 12 and 13, wherein the condensation component (B) is a 5-pyrazolone compound, a cresidine, phenetidine or N,N-di-lower alkylaniline compound, a 3-lower alkyl-6-di-lower alkylaminoindole compound, 2-lower alkylindole, 2-phenylindole, a 3-lower alkyl-6-lower alkoxyindole compound, or a 2-lower alkylindole, 2-phenylindole, 3-lower alkyl-6-lower alkoxyindole or 3-lower alkyl-6-di-lower alkylaminoindole compound each of which is N-substituted by C₁-C₈alkyl.

15. A recording material according to one of claims 12 to 14, wherein the colour-developing component (C) is a Lewis acid, an acid clay, a solid carboxylic acid or a compound containing a phenolic hydroxyl group.

16. A recording material according to one of claims 12 to 15 which is pressure-sensitive.

17. A recording material according to claim 16, wherein components (A) and (B) are dissolved in an organic solvent.

18. A recording material according to claim 17, wherein components (A) and (B) are encapsulated in microcapsules.

19. A recording material according to one of claims 16 to 18, wherein components (A) and (B) are present in the form of one or two layers on the back of a transfer sheet and component (C) is in the form of a layer on the face of a receiver sheet.

20. A recording material according to one of claims 12 to 15 which is heat-sensitive.

21. A recording material according to claim 20, which comprises 1 to 4 layer supports containing components (A), (B) and (C) and if desired a binder or wax or both.

22. A recording material according to one of claims 20 and 21, which additionally contains an activator.

23. A recording material according to one of claims 12 to 22, which contains the colour-forming components (A) and (B) together with one or more conventional colour formers.

24. A recording material according to claim 23, wherein the conventional colour former is a 3,3-(bisaminophenyl)phthalide, 3-indolyl-3-aminophenylaza- or -diazaphthalide, (3,3-bisindolyl)phthalide, 3-aminofluoran, 6-dialkylamino-2-dibenzylaminofluoran, 6-dialkylamino-3-methyl-2-arylaminofluoran, 3,6-bisalkoxyfluoran, 3,6-bis(diarylamino)fluoran, leucoauramine, spiropyran, spirodipyran, chromenopyrazole, chromenoindole, phenoxazine, phenothiazine, quinazoline, rhodamine lactam, carbazolylmethane or a further triarylmethane leuco dye.

## Revendications

1. Composés de types bis-phtalide-lactones de formule 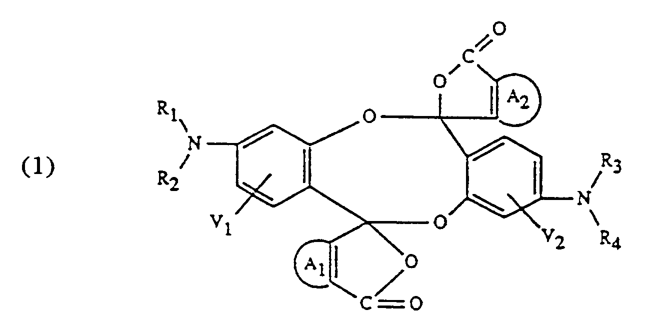 dans laquelle R₁, R₂, R₃ et R₄ représente indépendamment un atome d'hydrogène, un groupe alkyle comportant au plus 12 atomes de carbone substitué ou non par un atome d'halogène, un groupe hydroxy, cyano, tétrahydrofuryle ou alcoxy inférieur, un groupe acyle comportant de 1 à 12 atomes de carbone, un groupe cycloalkyle comportant de 5 à 10 atomes de carbone, ou un groupe aralkyle ou aryle substitué ou non par un atome d'halogène, un groupe cyano, nitro, trifluorométhyle, alkyle inférieur, alcoxy inférieur, alcoxycarbonyle inférieur, -NX'X'' ou 4-NX'X''-phénylamino, où X' et X''représentent indépendamment un atome d'hydrogène, un groupe alkyle inférieur, cyclohexyle, benzyle ou phényle, ou les paires de substituants (R₁ et R₂) et (R₃ et R₄) forment avec l'atome d'azote qu'ils ont en commun un hétérocycle à 5 ou 6 chaînons, V₁ et V₂ représentent un atome d'hydrogène ou d'halogène, un groupe alkyle inférieur, un groupe alcoxy en C₁₋₁₂, acyloxy en C₁₋₁₂, benzyle, phényle, benzyloxy, phényloxy, benzyle ou benzyloxy portant un substituant halogéno, cyano, alkyle inférieur ou alcoxy inférieur, ou le groupe -NT₁T₂, T₁ et T₂ représentant indépendamment un atome d'hydrogène, un groupe alkyle inférieur, un groupe cycloalkyle en C₅₋₁₀, un groupe benzyle substitué ou non par un atome d'halogène, un groupe cyano, alkyle ou alcoxy inférieurs, ou un groupe acyle comportant de 1 à 8 atomes de carbone et T₁ représente également un groupe phényle substitué ou non par un atome d'halogène, un groupe cyano, alkyle ou alcoxy inférieurs, et les cycles A₁ et A₂ représentent indépendamment un résidu aromatique ou hétérocyclique dont le cycle comporte 6 chaînons, et qui peut contenir un noyau aromatique condensé, les cycles A₁ et A₂ ainsi que les noyaux condensés pouvant être substitués.

2. Composés de type bis-phtalide-lactones conformes à la revendication 1, caractérisés en ce que, dans la formule (1), -NR₁R₂ et -NR₃R₄ sont identiques.

3. Composés de type bis-phtalide-lactones conformes à une des revendications 1 et 2, caractérisés en ce que, dans la formule (1), R₁, R₂, R₃ et R₄ représentent indépendamment des résidus alkyle inférieur, cyanoalkyle inférieur, cyclohexyle, tolyle ou benzyle ou -NR₁R₂ et -NR₃R₄ représentent des résidus pyrrolidino, N-(alkyle inférieur)-N-tétrahydrofurfurylamino, 4-di-(alkyle inférieur)-aminophénylamino ou 4-(4'-phénylamino-phénylamino)-phénylamino.

4. Composés de type bis-phtalide-lactones conformes à une des revendications 1 à 3, caractérisés en ce que, dans la formule (1), V₁ et V₂ représentent un atome d'hydrogène ou d'halogène, un résidu alkyle inférieur, alcoxy inférieur ou un groupe -NT₁T₂, un des résidus T₁ et T₂ représentant de préférence un groupe alkyle inférieur ou acyle en C₁₋₈ et l'autre un atome d'hydrogène ou un alkyle inférieur.

5. Composés de type bis-phtalide-lactones conformes à une des revendications 1 à 4, caractérisés en ce que, dans la formule (1), les noyaux A₁ et A₂ représente un noyau benzène, naphtalène, pyridine, pyrazine, quinoxaline ou quinoléine éventuellement substitués.

6. Composés de type bis-phtalide-lactones conformes à la revendications 1, caractérisés en ce qu'ils répondent à la formule 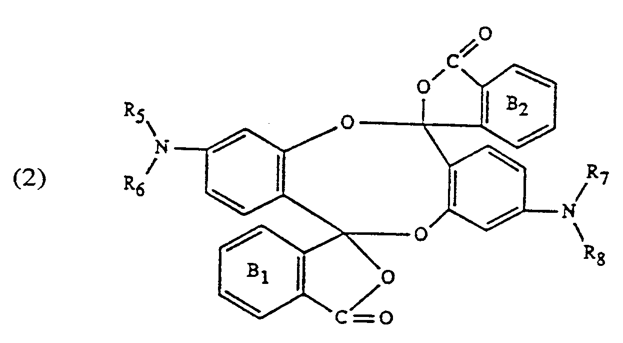 dans laquelle R₅, R₆, R₇ et R₈ représentent indépendemment des résidus alkyle en C₁₋₈, cycloalkyle en C₅₋₆ ou benzyle ou phényle non substitués ou portant des substituants halogéno, alkyle inférieur, alcoxy inférieur ou alcoxycarbonyle inférieur, ou les paires de substituants (R₅ et R₆) et (R₇ et R₈), représentent indépendamment, ensemble avec l'atome d'azote les reliant, un cycle pyrrolidino, pipéridino ou morpholino et les cycles B₁ et B₂ sont substitués ou portent un substituant halogéno, alkyle inférieur, alcoxycarbonyle inférieur ou dialkylamino inférieur.

7. Composés de type bis-phtalide-lactones conformes à la revendication 6, caractérisés en ce que, dans la formule (2), R₅, R₆, R₇ et R₈ représentent des résidus alkyle inférieurs ou benzyle, et les noyaux B₁ et B₂ sont non substitués ou portent des substituants halogéno, alkyle inférieur ou diméthylamino.

8. Composés de type bis-phtalide-lactones conformes à la revendication 1, caractérisés en ce qu'ils répondent à la formule 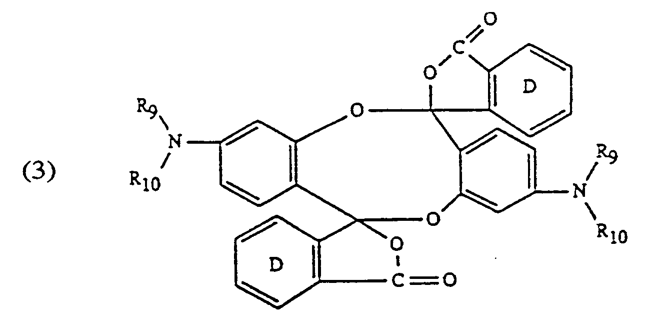 dans laquelle R₉ représente des résidus alkyle en C₁₋₆, cyclohexyle, benzyle, phényle ou des résidus benzyle ou phényle méthylés ou chlorés, et R₁₀représente un groupe alkyle en C₁₋₆, benzyle ou un résidu benzyle méthylé ou chloré et les noyaux D sont non substitués ou substitués par 1 à 4 atomes de chlore.

9. Composés de type bis-phtalide-lactones conformes à la revendication 8, caractérisés en ce que, dans la formule (3), R₉ et R₁₀ représentent un résidu alkyle en C₁₋₄ et les noyaux benzéniques D sont non substitués.

10. Procédé pour la préparation de composés de type bis-phtalide-lactones conformes à la revendication 1, caractérisé en ce que l'on fait réagir un acide cétonique de formule 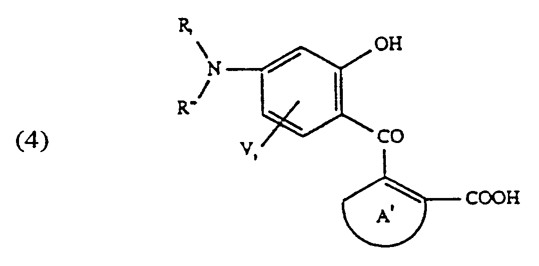 dans laquelle R', R'', V' et A' ont la signification de R₁, R₂, R₃, R₄, V₁, V₂, A₁ et A₂ conformément à la revendication 1, seul ou sous forme de mélange, en présence d'un agent de déshydratation.

11. Utilisation de composés de type bis-phtalide-lactones conformes à une des revendications 1 à 9 comme composants chromogènes dans un matériau d'enregistrement sensible à la pression ou à la chaleur.

12. Matériau d'enregistrement sensible à la chaleur ou à la pression, caractérisé en ce qu'il contient, dans son système de réactifs chromogènes, (A) un composé de type bis-phtalide-lactone conforme à une des revendications 1 à 9, (B) un composant de condensation organique et (C) un composant accepteur d'électrons, révélateur de couleur.

13. Matériau d'enregistrement conforme à la revendication 12, caractérisé en ce que le composant de condensation (B) est un composé de type aminophényle substitué sur l'atome d'azote, aminophénylstyrène substitué sur l'atome d'azote, acylacétarylamide, phénol mono- ou polyvalent, éther phénolique, éther 3-aminophénolique, aniline, naphtylamine, diarylamine, naphtol, naphtolcarbanilide, aminopyrimidine, aminopyrazol, pyrazolone, thiophène, thionaphtène, phénothiazine, aminothiazole, acridine, pyridone, indol, carbazole, kaïroline, indolizine, julotidine, morpholine, pyrrolidine, pipéridine, pipérazine, indoline, quinoléine, pyrimidone, acide barbiturique, benzomorpholine, dihydroquinoléine ou tétrahydroquinoléine.

14. Matériau d'enregistrement conforme à une des revendications 12 et 13, caractérisé en ce que le composant de condensation (B) est un composé de type 5-pyrazolone, crésidine, phénétidine ou N,N-di-(alkyle inférieur)aniline, 3-(alkyle inférieur)-6-di(alkyle inférieur)-aminoindole, 2-(alkyle inférieur)-indole, 2-phénylindoles, 3-(alkyle inférieur)-6-(alcoxy inférieur), ou encore 2-(alkyle inférieur)-indole, 2-phénylindole, 3-(alkyle inférieur)-6-(alcoxy inférieur) ou 3-(alkyle inférieur)-6-di(alkyle inférieur)aminoindole substitués sur l'atome d'azote par un groupe alkyle en C₁₋₈.

15. Matériau d'enregistrement conforme à une des revendications 12 à 14, caractérisé en ce que le composant révélateur (C) est un acide de Lewis, une argile acide, un acide carboxylique solide ou un composé à groupe hydroxyle phénolique.

16. Matériau d'enregistrement conforme à une des revendications 12 à 15, caractérisé en ce qu'il est sensible à la pression.

17. Matériau d'enregistrement conforme à la revendication 16, caractérisé en ce que les composants (A) et (B) sont dissous dans un solvant organique.

18. Matériau d'enregistrement conforme à la revendication 17, caractérisé en ce que les composants (A) et (B) sont encapsulés dans des microcapsules.

19. Matériau d'enregistrement conforme à une des revendications 16 à 18, caractérisé en ce que les composants (A) et (B) sont sous forme d'une ou de deux couches sur le côté verso d'une feuille de transfert et le composant (C) est sous forme d'une couche sur le côté recto d'une feuille réceptrice.

20. Matériau d'enregistrement conforme à une des revendications 12 à 15, caractérisé en ce qu'il est thermosensible.

21. Matériau d'enregistrement conforme à la revendication 20, caractérisé en ce qu'il comprend de 1 à 4 couches de support dans lesquelles sont présents les composants (A), (B) et (C) et éventuellement un liant et/ou une cire.

22. Matériau d'enregistrement conforme à une des revendications 20 et 21, caractérisé en ce qu'il contient en outre un activateur.

23. Matériau d'enregistrement conforme à une des revendications 12 à 22, caractérisé en ce que les composants chromogènes (A) et (B) sont présents en combinaison avec un ou plusieurs agents chromogènes conventionnels.

24. Matériau d'enregistrement conforme à la revendication 23, caractérisé en ce qu'il contient comme composant chromogène conventionnel des 3,3-(bisaminophényl)-phtalides, 3-indolyl-3-aminophénylaza- ou diazaphtalides, (3,3-bis-indolyl)-phtalides, 3-aminofluoranes, 6-dialkylamino-2-dibenzylaminofluoranes, 6-dialkylamino-3-méthyl-2-arylaminofluoranes, 3,6-bisalcoxyfluoranes, 3,6-bis-diarylaminofluoranes, leucoauramines, spiropyranes, spirodipyranes, chroménopyrazoles, chroménoindoles, phénoxazines, phénothiazines, quinazolines, rhodaminelactames, carbazolylméthanes ou les formes leuco d'autres colorants de type triarylméthane.
